# EUROPÄISCHE PATENTANMELDUNG

(11) **EP 3 643 345 A1**
(43) Veröffentlichungstag der Anmeldung: **29.04.2020**
(21) Anmeldenummer: 18201924.0
(22) Anmeldetag: 23.10.2018
(51) Int. Cl.: A61M 5/158, A61M 25/02

(54) **INFUSIONSSET FÜR EINE INFUSIONSVORRICHTUNG**

(71) Anmelder: Tecpharma Licensing AG, 3401 Burgdorf (CH)
(72) Erfinder: Marggi, Rolf, 3006 Bern (CH); Renggli, Christoph, 2540 Grenchen (CH); Zurflüh, Andreas, 3415 Hasle-Rüegsau (CH)

(57) **Zusammenfassung**

Die Erfindung betrifft ein Infusionsset mit
- einem Basisteil (1; 1'), wobei der Basisteil mit einem Kappenteil (3; 3') zum Halten des Infusionssets durch einen Patienten oder durch eine Insertionsvorrichtung oder Einstechhilfe verbindbar ist oder mit einem Konnektorteil (2, 2') mit einer Zuführleitung zur Verbindung mit einer Infusionsvorrichtung verbindbar ist, wobei an einer distalen Seite des Basisteils (1, 1') eine Kanüle (1a; 1b) zur Zuführung eines flüssigen Medikaments in den Körper des Patienten vorgesehen ist und wobei ferner an der distalen Seite eine Nocke (1h, 1h'), insbesondere mehrere Nocken (1h, 1h') vorgesehen sind,
-einem Heftpflaster (1c, 1c') zur Befestigung des Infusionssets auf der Haut des Patienten, wobei die Nocke (1h, 1h'), insbesondere die mehreren Nocken (1h, 1h') mit dem Heftpflaster verbunden, insbesondere verschmolzen sind.

## Beschreibung

Die Erfindung betrifft ein Infusionsset für eine Infusionsvorrichtung. Infusionssets dienen der Zuführung eines flüssigen Produkts, insbesondere eines flüssigen Medikaments von einer Infusionsvorrichtung in den Körper eines Patienten.

Der Begriff "Medikament" umfasst hier jede fliessfähige medizinische Formulierung, welche geeignet ist zur kontrollierten Verabreichung durch ein Mittel, wie z. B. eine Kanüle oder eine Hohlnadel, hindurch, beispielsweise umfassend eine Flüssigkeit, eine Lösung, ein Gel oder eine feine Suspension, welche(s) einen oder mehrere medizinische Wirkstoffe enthält. Medikament kann eine Zusammensetzung mit einem einzigen Wirkstoff oder eine vorgemischte oder co-formulierte Zusammensetzung mit mehreren Wirkstoffen aus einem einzelnen Behälter sein. Medikament umfasst Arzneien wie Peptide (z.B. Insuline, Insulin enthaltende Medikamente, GLP-1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzyme und weitere Substanzen sowohl in fester (suspendierter) oder flüssiger Form aber auch Polysaccharide, Vakzine, DNS oder RNS oder Oligonukleotide, Antikörper oder Teile von Antikörpern sowie geeignete Basis-, Hilfs- und Trägerstoffe.

Bei Patienten, welche einen regelmässigen Bedarf an einem flüssigen Medikament haben, kann dem Körper die erforderliche Medikamentenmenge in flüssiger Form mittels einer über einen längeren Zeitraum im subkutanen Gewebe angeordneten Kanüle zugeführt werden. Hierzu wird auf der Haut des Patienten ein Infusionsset mit einer Kanüle aus einem weichen Material oder aus einem steifen Material derart befestigt, dass die Kanüle durch die Haut bis in das subkutane Gewebe eindringt. Die Verabreichung der erforderlichen Medikamentenmenge in flüssiger Form erfolgt somit von einer Infusionsvorrichtung, insbesondere von einer Infusionspumpe, besonders bevorzugt von einer automatischen Infusionspumpe über eine Zuführleitung zum Patienten, wobei die in dem Infusionsset angeordnete Kanüle den Patienten mit dem flüssigen Medikament versorgt.

Aus dem Stand der Technik sind Infusionssets bekannt. Die Dokumente US8152769B2, EP1694385B1 und EP1984044B1 offenbaren Infusionssets, welche einen Basisteil mit einer Kanüle aufweisen, wobei die Kanüle zum Eindringen durch die Haut bis in das subkutane Gewebe dient, um ein flüssiges Medikament an einen Patient zu verabreichen. Ferner weisen die Infusionssets einen Konnektorteil auf, an welchem eine Zuführleitung, insbesondere ein Schlauch angebracht ist. Die Zuführleitung kann mit einer Infusionsvorrichtung, insbesondere mit einer Infusionspumpe verbunden werden. Somit kann durch das Infusionsset das flüssige Medikament von der Infusionsvorrichtung dem Patienten verabreicht werden. Der Konnektorteil und der Basisteil können relativ zueinander drehbar sein, insbesondere um einen beliebigen Winkel relativ zueinander drehbar sein. Durch diese Anordnung kann der Tragkomfort verbessert werden. Nachteilig bei diesen Infusionssets ist, dass die Handhabung oder/und die Bedienung dieser Infusionssets erschwert sind, da Fehlbedienungen möglich sind.

Es ist eine Aufgabe der Erfindung ein alternatives Infusionsset bereitzustellen, wobei die Handhabung und/oder die Bedienung des Infusionssets erleichtert sind, um Fehlbedienungen zu verhindern.

Diese Aufgabe wird durch den Gegenstand der unabhängigen Ansprüche gelöst. Vorteilhafte Ausführungsformen sind in den abhängigen Ansprüchen definiert.

In die distale Richtung bedeutet bei dem erfindungsgemässen Infusionsset die Seite, an welcher ein Heftpflaster angebracht sein kann, um das Infusionsset auf der Haut eines Patienten anzubringen. In die proximale Richtung bedeutet bei dem erfindungsmässen Infusionsset die Seite, die mit einer Infusionsvorrichtung verbunden werden kann.

Das erfindungsgemässe Infusionsset kann an eine Infusionsvorrichtung, insbesondere an eine Infusionspumpe, besonders bevorzugt an eine automatische Infusionspumpe anbringbar sein. Infusionsvorrichtungen können der kontinuierlichen Verabreichung von Infusionen, insbesondere von Medikamenten in flüssiger Form dienen. Bei der Infusionsvorrichtung kann es sich um eine wiederverwendbare Infusionsvorrichtung handeln, wobei ein neuer mit einem flüssigen Medikament vorgefüllter Container in die wiederverwendbare Infusionsvorrichtung einsetzbar ist. Andererseits kann es sich bei der Infusionsvorrichtung um eine einmalig verwendbare Infusionsvorrichtung handeln, wobei die Infusionsvorrichtung weggeworfen wird, wenn ein mit einem flüssigen Medikament vorgefüllter Container leer ist. Das Infusionsset kann einen Basisteil zum Anbringen auf der Haut eines Patienten umfassen, wobei der Basisteil eine proximale Seite und eine distale Seite aufweisen kann. Ferner kann der Basisteil eine Kanüle zur Zuführung des flüssigen Medikaments in den Körper des Patienten aufweisen. Die Kanüle kann auf der distalen Seite des Basisteils vorgesehen sein. Ferner kann das Infusionsset einen Konnektorteil mit einer Zuführleitung, insbesondere einem Schlauch umfassen. Der Konnektorteil kann eine proximale Seite und eine distale Seite aufweisen. Die Zuführleitung kann zur Verbindung mit der Infusionsvorrichtung dienen. Ferner kann die Zuführleitung mittels einer Durchstechkanüle zur Verbindung mit dem Basisteil dienen, um einen Fluidpfad zwischen dem Konnektorteil und dem Basisteil zu bilden. Ferner kann das Infusionsset einen Kappenteil zum Halten des Infusionssets durch den Patienten oder durch eine Insertionsvorrichtung oder Einstechhilfe umfassen, wobei der Kappenteil eine distale Seite und eine proximale Seite aufweisen kann. Das Infusionsset kann in einer ersten Ausführungsform mit einer weichen Kanüle, wobei die Kanüle aus einem weichen Material, insbesondere aus einem Polymer ausgebildet sein kann, oder in einer zweiten Ausführungsform mit einer steifen Kanüle, wobei die Kanüle aus Stahl oder aus einem alternativen steifen Material ausgebildet sein kann, ausgestattet sein.

In einer ersten Ausführungsform des Infusionssets kann der Kappenteil des Infusionssets eine steife Nadel, wobei die Nadel aus Stahl oder aus einem alternativen steifen Material ausgebildet sein kann, aufweisen. Der Kappenteil kann mit dem Basisteil lösbar verbunden werden oder sein. Die Verbindung kann vorzugsweise als Schnappverbindung ausgebildet sein. Alternative Verbindungen können vorgesehen sein. Der Kappenteil kann mit dem Basisteil derart verbunden, insbesondere verschnappt sein, dass die steife Nadel konzentrisch in einer weichen Kanüle des Basisteils angeordnet sein kann. Die weiche Kanüle kann der Zuführung eines flüssigen Medikaments in den Körper eines Patienten dienen. Der Kappenteil kann an der proximalen Seite des Basisteils angeordnet werden oder sein. Die konzentrische Anordnung der weichen Kanüle und der steifen Nadel kann der Führung der weichen Kanüle dienen, wenn die weiche Kanüle des Basisteils in die Haut des Patienten eingestochen wird. Dabei kann die distale Spitze der steifen Nadel über das distale Ende der weichen Kanüle ragen. Ferner kann an der distalen Seite des Basisteils ein Heftpflaster angeordnet sein. Das Heftpflaster kann dem Befestigen des Infusionssets auf der Haut des Patienten dienen. Um das Infusionsset auf der Haut des Patienten anzubringen, kann somit der mit dem Kappenteil verbundene, insbesondere verschnappte Basisteil mittels Heftpflaster auf die Haut des Patienten geklebt werden, wobei vorgängig eine Schutzfolie von einer Klebeseite des Heftpflasters entfernt werden kann. Dabei kann die mit der festen Nadel aufgenommene weiche Kanüle durch die Haut des Patienten und in das subkutane Geweben des Patienten eindringen. Um das Infusionsset mit einer Infusionsvorrichtung zu verbinden, kann zuerst der Kappenteil mit der Nadel von dem Basisteil abgenommen werden und ein Konnektorteil, welcher eine Zuführleitung, insbesondere einen Schlauch und einen Adapter umfassen kann, an den Basisteil des Infusionssets angebracht werden. Die Verbindung zwischen dem Basisteil und dem Konnektorteil kann als Schnappverbindung ausgebildet sein. Alternative Verbindungen können vorgesehen sein. Ein erstes Ende der Zuführleitung kann mit dem Adapter verschweisst und/oder verklebt sein. Ein zweites Ende der Zuführleitung kann mit dem Konnektorteil verschweisst und/oder verklebt sein. Besonders bevorzugt kann die Zuführleitung mit dem Adapter und/oder mit dem Konnektorteil verklebt sein, vorzugsweise mit einem Klebstoff, der durch Bestrahlung mit UV-Licht aushärten kann. Dadurch kann auf Lösungsmittel, welche beim Verschweissen verwendet werden, verzichtet werden. Die Verwendung von Lösungsmittel kann unerwünscht sein, da ein Lösungsmittel in den Fluidpfad gelangen könnte und somit das abzugebende flüssige Medikament kontaminieren könnte. Der Adapter des Konnektorteils kann lösbar an ein entsprechendes korrespondierendes Verbindungselement der Infusionsvorrichtung angebracht werden. Der Adapter des Konnektorteils kann mittels Bajonettverschluss, Luerverschluss oder einem anderen Verschluss mit der Injektionsvorrichtung verbunden werden. Somit kann ein Fluidpfad von der Infusionsvorrichtung über das Infusionsset zu dem Patienten entstehen.

Der Adapter des Konnektorteils kann ein Ventil umfassen. Das Ventil kann derart ausgestaltet sein, dass sich das Ventil öffnen und schliessen kann. Das Ventil kann als Rückschlagventil, insbesondere als Membran-Rückschlagventil ausgebildet sein. Das Ventil kann einen Flüssigkeitszulauf und einen Flüssigkeitsablauf für das flüssige Medikament umfassen.

Das Ventil kann ferner derart ausgestaltet sein, dass es sich öffnen kann, wenn der Druck des flüssigen Medikaments in dem Durchflusszulauf einen zuvor bestimmten Schwellenwert überschreitet. Dadurch kann eine unkontrollierte Abgabe von dem flüssigen Medikament verhindert werden.

Das Ventil kann ferner derart ausgestaltet sein, dass das flüssige Medikament nur in eine Richtung von dem Flüssigkeitszulauf zu dem Flüssigkeitsablauf fliessen kann. Dadurch kann ein Rückfluss des abzugebenden flüssigen Medikaments verhindert werden.

In einer zweiten Ausführungsform des Infusionssets kann das Infusionsset einen Kappenteil umfassen. Der Basisteil kann eine steife Kanüle umfassen. Die steife Kanüle kann der Zuführung eines flüssigen Medikaments in den Körper eines Patienten dienen. Der Kappenteil kann mit dem Basisteil lösbar verbunden werden oder sein. Die Verbindung kann vorzugsweise als Schnappverbindung ausgebildet sein. Alternative Verbindungen können vorgesehen sein. Der Basisteil kann an dem distalen Ende des Kappenteils angeordnet werden oder sein. An der distalen Seite des Basisteils kann ein Heftpflaster zur Befestigung des Infusionssets auf der Haut des Patienten vorgesehen sein. Um das Infusionsset auf der Haut des Patienten anzubringen, kann der mit dem Kappenteil verbundene, insbesondere verschnappte Basisteil mittels Heftpflaster auf die Haut des Patienten geklebt werden, wobei vorgängig eine Schutzfolie von einer Klebeseite des Heftpflasters entwerft werden kann. Dabei kann die steife Kanüle des Basisteils in das subkutane Gewebe des Patienten eindringen. Danach kann der Kappenteil von dem Basisteil entfernt werden, um ein Konnektorteil mit dem Basisteil lösbar zu verbinden. Der Konnektorteil kann eine Zuführleitung, insbesondere einen Schlauch und einen Adapter, um das Infusionsset mit einer Infusionsverbindung lösbar zu verbinden, umfassen. Die Verbindung zwischen dem Basisteil und dem Konnektorteil kann als Schnappverbindung ausgebildet sein. Alternative Verbindungen können vorgesehen sein. Die Zuführleitung kann mit einem ersten Ende mit dem Adapter verschweisst und/oder verklebt sein und mit einem zweiten Ende mit dem Konnektorteil verschweisst und/oder verklebt sein. Besonders bevorzugt kann die Zuführleitung mit dem Adapter und/oder mit dem Konnektorteil verklebt sein, vorzugsweise mit einem Klebstoff, der durch Bestrahlung mit UV-Licht aushärten kann. Dadurch kann auf Lösungsmittel, welche beim Verschweissen verwendet werden, verzichtet werden. Die Verwendung von Lösungsmittel kann unerwünscht sein, da ein Lösungsmittel in den Fluidpfad gelangen könnte und somit das abzugebende flüssige Medikament kontaminieren könnte. Der Adapter des Konnektorteils kann lösbar an ein entsprechendes korrespondierendes Verbindungselement der Infusionsvorrichtung angebracht werden.

Der Adapter des Konnektorteils kann mittels Bajonettverschluss, Luerverschluss oder einem anderen Verschluss mit der Injektionsvorrichtung verbunden werden. Somit kann ein Fluidpfad von der Infusionsvorrichtung über das Infusionsset zu dem Patienten entstehen.

Der Adapter des Konnektorteils kann ein Ventil umfassen. Das Ventil kann derart ausgestaltet sein, dass sich das Ventil öffnen und schliessen kann. Das Ventil kann als Rückschlagventil, insbesondere als Membran-Rückschlagventil ausgebildet sein. Das Ventil kann einen Flüssigkeitszulauf und einen Flüssigkeitsablauf für das flüssige Medikament umfassen.

Das Ventil kann ferner derart ausgestaltet sein, dass es sich öffnen kann, wenn der Druck des flüssigen Medikaments in dem Durchflusszulauf einen zuvor bestimmten Schwellenwert überschreitet. Dadurch kann eine unkontrollierte Abgabe von dem flüssigen Medikament verhindert werden.

Das Ventil kann ferner derart ausgestaltet sein, dass das flüssige Medikament nur in eine Richtung von dem Flüssigkeitszulauf zu dem Flüssigkeitsablauf fliessen kann. Dadurch kann ein Rückfluss des abzugebenden flüssigen Medikaments verhindert werden.

Die erste und die zweite Ausführungsform des Infusionssets können vorzugsweise ein lösbares Schutzelement aufweisen. Das Schutzelement kann dem Schutz vor Stichverletzungen durch die steife Kanüle oder durch die steife Nadel des entsprechenden Infusionssets dienen. Das Schutzelement kann vorzugsweise hülsenförmig ausgebildet sein. Alternative Ausgestaltungen des Schutzelements können vorgesehen sein. An einer Mantelaussenfläche des Schutzelements können vorzugsweise eine oder mehrere Längsrippen zum besseren Greifen des Schutzelements vorgesehen sein. Somit kann der Patient vor dem Anbringen des Infusionssets auf der Haut des Patienten das Schutzelement einfach von dem Basisteil abnehmen und nach dem Entfernen des Infusionssets von der Haut des Patienten wieder einfach über die steife Kanüle des Basisteils oder über die steife Nadel des Kappenteils aufsetzen, wobei der Patient somit vor Stichverletzungen geschützt sein kann. Das Schutzelement kann mit einem Kraftschluss und/oder mit einem Formschluss mit dem Basisteil und/oder dem Kappenteil lösbar verbunden sein, um den Patienten vor einer Stichverletzung zu schützen.

Der Basisteil der ersten und der zweiten Ausführungsform des Infusionssets kann vorzugsweise flach ausgebildet sein. Zur Führung und/oder zur Positionierung des Basisteils relativ zu dem Kappenteil und/oder zu dem Konnektorteil kann auf der proximalen Seite des Basisteils der ersten und der zweiten Ausführungsform des Infusionssets ein Basiszylinder vorgesehen sein. Der Basiszylinder kann von der proximalen Seite des Basisteils in die proximale Richtung abragen. Der Basiszylinder kann zylinderförmig ausgebildet sein. Der Basiszylinder kann konzentrisch zu der weichen oder zu der steifen Kanüle des entsprechenden Infusionssets angeordnet sein.

Ferner kann in dem Basisteil der ersten und der zweiten Ausführungsform des Infusionssets eine Öffnung vorgesehen sein. Die Öffnung kann als Blindloch ausgebildet sein. Die Öffnung kann auf der distalen Seite des Basisteils angeordnet sein und kann in dem Basiszylinder vorgesehen sein. Die Öffnung kann konzentrisch zu der weichen oder zu der steifen Kanüle des entsprechenden Infusionssets angeordnet sein. Die Öffnung kann derart ausgestaltet sein, dass die Öffnung das Schutzelement aufnehmen, insbesondere kraftschlüssig aufnehmen kann. Alternativ können die Öffnung und das Schutzelement derart ausgestaltet sein, dass das Schutzelement formschlüssig in der Öffnung aufgenommen werden kann.

Ferner kann an dem Basisteil der ersten und der zweiten Ausführungsform des Infusionssets vorzugsweise ein Kanülenzylinder an der distalen Seite des Basisteils vorgesehen sein. Der Kanülenzylinder kann zylinderförmig ausgebildet sein. Der Kanülenzylinder kann in der Öffnung des Basisteils vorgesehen sein. Der Kanülenzylinder kann von der distalen Seite des Basisteils in die distale Richtung abragen. Die weiche oder die steife Kanüle kann in dem entsprechenden Kanülenzylinder, insbesondere in einem in dem Kanülenzylinder vorgesehenen Durchgang befestigt sein und von dem entsprechenden Kanülenzylinder in die distale Richtung abragen, um das flüssige Medikament dem Patienten abzugeben.

Vor dem Anbringen des entsprechenden Infusionssets auf der Haut des Patienten, kann das Schutzelement mit einem Kraftschluss mit dem Basisteil lösbar verbunden sein, damit das Schutzelement den Patienten vor einer Stichverletzung durch die steife Kanüle oder durch die steife Nadel des entsprechenden Infusionssets schützen kann. Das Schutzelement kann derart in der Öffnung des Basisteils lösbar eingepresst oder einpressbar sein, dass das Schutzelement auf Kompression belastet ist. Die Druckkraft auf das Schutzelement kann zu weniger Schäden, insbesondere Risse an dem Schutzelement führen, als wenn das Schutzelement auf dem Kanülenzylinder des Basisteils angeordnet ist und somit auf Zug belastet ist. Ferner kann der Durchmesser des Schutzelements grösser sein, sodass der Patient das Schutzelement besser greifen kann, um das Schutzelement von dem Basisteil wegzunehmen und/oder auf den Basisteil aufzusetzen. Durch diese Anordnung kann ferner der Druck auf den Kanülenzylinder reduziert werden, sodass der Kanülenzylinder nicht, beispielsweise durch Risse oder Dellen beschädigt wird.

Alternativ kann das Basisteil eine oder mehrere Ausnehmungen aufweisen, wobei ein oder mehrere an dem Schutzelement vorgesehene stiftförmige Elemente in die Ausnehmung oder die mehreren Ausnehmungen lösbar eingepresst oder einpressbar sind, um den Patienten vor Stichverletzungen zu schützen. Alternativ kann die Ausnehmung oder die mehreren Ausnehmungen und das stiftförmige Element oder die mehreren stiftförmigen Elemente derart ausgestaltet sein, dass die Ausnehmung oder die mehreren Ausnehmungen das stiftförmige Element oder die mehreren stiftförmigen Element formschlüssig aufnehmen können.

Das Kappenteil der ersten und der zweiten Ausführungsform des Infusionssets kann vorzugsweise kappenförmig ausgebildet sein. An der Mantelinnenseite des Kappenteils kann ein Kappenzylinder vorgesehen sein. Der Kappenzylinder kann zylinderförmig ausgebildet sein. Der Kappenzylinder kann einteilig oder mehrteilig ausgebildet sein. Der Kappenzylinder kann hohlzylinderförmig ausgebildet sein. Der Kappenzylinder kann sich von der Mantelinnenseite in die distale Richtung erstrecken. Der Kappenzylinder kann derart an dem Kappenteil angeordnet sein, dass wenn der Kappenteil mit dem Basisteil verbunden, insbesondere verschnappt ist, der Kappenzylinder konzentrisch zu dem Basiszylinder des Basisteils angeordnet sein kann. Wenn der Kappenteil mit dem Basisteil verbunden, insbesondere verschnappt wird oder von dem Basisteil weggenommen wird, kann der Basiszylinder mit dem Kappenzylinder in einem Führungseingriff, insbesondere in einem Gleiteingriff sein. Der Kappenzylinder des Kappenteils kann ausserhalb des Basiszylinders des Basisteils angeordnet sein. Der Kappenteil kann beim Verbinden, insbesondere Verschnappen mit dem Basisteil und/oder Lösen von dem Basisteil axial zu dem Basisteil bewegbar angeordnet sein.

Der Konnektorteil der ersten und der zweiten Ausführungsform des Infusionssets kann vorzugsweise kappenförmig ausgebildet sein. Ferner kann an der Mantelinnenseite des Konnektorteils ein Konnektorzylinder vorgesehen sein. Der Konnektorzylinder kann zylinderförmig ausgebildet sein. Der Konnektorzylinder kann sich von der Mantelinnenseite in die distale Richtung erstrecken. Der Konnektorzylinder kann derart an dem Konnektorteil angeordnet sein, dass wenn der Konnektorteil mit dem Basisteil verbunden, insbesondere verschnappt ist, der Konnektorzylinder konzentrisch zu dem Kanülenzylinder des Basisteils angeordnet sein kann. Der Konnektorzylinder des Konnektorteils kann ausserhalb des Basiszylinders des Basisteils angeordnet sein. Der Konnektorzylinder kann einteilig oder mehrteilig ausgebildet sein. Der Konnektorzylinder kann hohlzylinderförmig ausgebildet sein, wobei im Innern des Konnektorzylinders eine Durchstechkanüle konzentrisch und/oder koaxial zu dem Konnektorzylinder angeordnet sein kann. Die Durchstechkanüle kann als separates Teil, insbesondere als separate Stahlkanüle oder als Bestandteil des Konnektorteils ausgebildet sein. Die Durchstechkanüle kann mit der Zuführleitung, insbesondere mit dem Schlauch verbunden sein. Wenn der Konnektorteil mit dem Basisteil verbunden, insbesondere verschnappt wird oder von dem Basisteil weggenommen wird, kann der Basiszylinder in einem Führungseingriff, insbesondere in einem Gleiteingriff mit dem Konnektorzylinder sein. Wenn der Konnektorzylinder konzentrisch zu dem Basiszylinder angeordnet ist, kann sich der Konnektorteil relativ zu dem Basisteil drehen, insbesondere um einen beliebigen Winkel drehen. Der Konnektorteil und der Basisteil können relativ zueinander axial bewegbar und drehbar, insbesondere um einen beliebigen Winkel drehbar angeordnet sein.

In der ersten Ausführungsform des Infusionssets kann die steife Kanüle mit der korrekten Länge in den Basisteil, insbesondere in den Kanülenzylinder, insbesondere in den Durchgang des Kanülenzylinders des Basisteils eingesetzt werden und ein Klebstoff kann zur Befestigung der steifen Kanüle in dem Basisteil ausserhalb des Fluidpfads, insbesondere zwischen der steifen Kanüle und dem Kanülenzylinder des Basisteils angebracht werden. Somit kann die Kontaminierung des abzugebenden flüssigen Medikaments durch den Klebstoff ausgeschlossen werden. Vorzugsweise kann ein Klebstoff verwendet werden, der durch Bestrahlung mit UV-Licht aushärten kann, um auf Lösungsmittel zu verzichten. Alternative Befestigungsmittel zur Befestigung der steifen Kanüle an dem Basisteil können vorgesehen sein.

In der zweiten Ausführungsform des Infusionssets kann die weiche Kanüle mit der korrekten Länge in den Basisteil, insbesondere in den Kanülenzylinder des Basisteils eingesetzt werden oder im eingesetzten Zustand auf die korrekte Länge zugeschnitten werden. Zur Verbindung, insbesondere zur festen Verbindung der weichen Kanüle in dem Basisteil kann ein Verbindungsteil verwendet werden. Der Verbindungsteil kann vorzugsweise an dem distalen Ende rohrförmig und an dem proximalen Ende trichterförmig ausgebildet sein. Andere Gestaltungsformen des Verbindungsteils können vorgesehen sein. Der Verbindungsteil kann aus steifem Material, insbesondere aus Stahl ausgebildet sein. Der Verbindungsteil kann als "eyelet" bezeichnet werden. Das "eyelet" kann der Führung der steifen Nadel dienen, welche an dem Kappenteil angeordnet ist. Um die weiche Kanüle in den Basisteil einsetzen zu können, kann vorerst die weiche Kanüle auf oder über das "eyelet", insbesondere über den distalen Teil des "eyelet" angebracht oder geschoben werden. Die weiche Kanüle kann mit dem "eyelet" kraftschlüssig verbunden sein insbesondere verpresst sein. Das mit der weichen Kanüle verbundene "eyelet" kann danach in den an dem Kanülenzylinder vorgesehenen Durchgang eingepresst werden. Das mit der weichen Kanüle verbundene "eyelet" kann mit dem Basisteil kraftschlüssig verbunden sein. Der proximale Teil des "eyelet" kann auf der proximalen Seite des Kanülenzylinders und der distale Teil des "eyelet" kann auf der distalen Seite des Kanülenzylinders angeordnet sein.

Ferner kann in dem Kanülenzylinder der ersten und der zweiten Ausführungsform des Infusionssets ein Septum angeordnet sein. In der ersten Ausführungsform des Infusionssets kann das Septum an dem proximalen Ende der steifen Kanüle zum Verschluss des Durchgangs des Kanülenzylinders angeordnet sein. In der zweiten Ausführungsform des Infusionssets kann das Septum an dem proximalen Ende des "eyelet" zum Verschluss des Durchgangs des Kanülenzylinders vorgesehen sein. Das Septum kann dem Verschluss des Basisteils zur Verhinderung von unerwünschtem Fluidfluss dienen. Die Durchstechkanüle des Konnektorteils kann das Septum des Basisteils durchstechen, insbesondere wenn der Konnektorteil mit dem Basisteil verbunden, insbesondere verschnappt ist, um eine fluide Verbindung zu bilden.

Die Zuführleitung, insbesondere der Schlauch der ersten und der zweiten Ausführungsform des Infusionssets kann vorzugsweise ein äusseres Material und ein inneres Material umfassen. Besonders bevorzugt kann das äussere Material ein Polyurethan (PU), insbesondere Elastollan ® 1185 A 10FC umfassen. Das innere Material kann besonders bevorzugt ein High Density Polyethylen (HDPE), insbesondere SABIC ® HDPE B5429 oder ein Polypropylen (PP), insbesondere Bormed TM SC820CF-11 umfassen. Ein alternatives äusseres und/oder inneres Material kann vorgesehen sein.

In einer Ausführungsform der Zuführleitung, insbesondere des Schlauchs kann die Zuführleitung, insbesondere der Schlauch des Infusionssets aus einem äusseren und einem inneren Material bestehen. Besonders bevorzugt kann das äussere Material aus einem Polyurethan (PU), insbesondere Elastollan ® 1185 A 10FC bestehen. Das innere Material kann besonders bevorzugt aus einem High Density Polyethylen (HDPE), insbesondere SABIC ® HDPE B5429 oder ein Polypropylen (PP), insbesondere Bormed TM SC820CF-11 bestehen. Der zweischichtige Schlauch kann durch ein Ko-Extrusionsverfahren hergestellt werden, danach in einem Wasserbad abgekühlt, auf eine Rolle aufgerollt werden und danach auf eine entsprechende Länge geschnitten werden. Die Haftung der zwei Schichten kann während dem Ko-Extrusionsverfahren durch einen chemischen und/oder physikalischen Vorgang entstehen. Der zweischichtige Schlauch kann keine Haftschicht zwischen den beiden Schichten umfassen. Durch den Verzicht einer Haftschicht zwischen den beiden Schichten kann verhindert werden, dass das abzugebende flüssige Medikament durch ein in der Haftschicht aufgenommenen Stoff kontaminiert wird.

In einer Ausführungsform der Zuführleitung, insbesondere des Schlauchs kann der Aussendurchmesser des äusseren Materials etwa zwischen 1,47mm und 1,57mm sein. Der Innendurchmesser des äusseren Materials und der Aussendurchmesser des inneren Materials kann etwa 0,6mm sein. Der Innendurchmesser des inneren Materials kann etwa zwischen 0,26mm und 0,34mm sein.

In einer anderen Ausführungsform der Zuführleitung, insbesondere des Schlauches kann der Aussendurchmesser des äusseren Materials etwa zwischen 1,47mm und 1,57mm sein. Der Innendurchmesser des äusseren Materials und der Aussendurchmesser des inneren Materials kann etwa 0,8mm sein. Der Innendurchmesser des inneren Materials kann etwa zwischen 0,46mm und 0,54mm sein. Die Wanddicke des inneren Materials kann derart verkleinert sein, dass weniger Volumen des inneren Materials zur Herstellung des Schlauches verwendet werden kann. Somit kann weniger Konservierungsmittel, beispielsweise Phenol oder m-Kresol von dem inneren Material des Schlauchs absorbiert werden und somit kann die Haltbarkeit des durch den Schlauch abzugebenden flüssigen Medikaments erhöht werden.

Die Materialwahl des äusseren Materials kann vorzugsweise eine gute Zugfestigkeit bzw. eine gute Reissfestigkeit, eine Hydrolysebeständigkeit, eine Kälteflexibilität und/oder eine Resistenz gegen Mikroorganismen aufweisen.

Der Basisteil der ersten und der zweiten Ausführungsform kann auf der distalen Seite eine unebene Fläche aufweisen. An der distalen Seite des Basisteils kann vorzugsweise eine, insbesondere mehrere Nocken vorgesehen sein. Die mehreren Nocken können auf der distalen Seite des Basisteils gleichmässig verteilt angeordnet sein. Alternativ können die mehreren Nocken auf der distalen Seite des Basisteils unregelmässig verteilt angeordnet sein. Die Nocken können vorzugsweise etwa pyramidenförmig oder kegelförmig ausgebildet sein. Die Nocken können vorzugsweise mit dem Heftpflaster verbunden sein, insbesondere verschmolzen sein. Vorzugsweise können die Nocken des Basisteils durch ein Ultraschallschweissen mittels einer Sonotrode mit dem Heftpflaster verschmolzen sein. Diese Anordnung kann, wenn der Basisteil auf der Haut des Patienten angebracht ist, ein verbessertes Belüften der weichen oder der steifen Kanüle des Infusionssets in der Haut des Patienten bewirken, da Luft zwischen dem Basisteil und dem Heftpflaster zirkulieren kann.

Der Basisteil der ersten und der zweiten Ausführungsform kann vorzugsweise eine Ausnehmung aufweisen. Die Ausnehmung kann an der proximalen Seite des Basisteils vorgesehen sein. Besonders bevorzugt können mehrere Ausnehmungen vorgesehen sein. Die Ausnehmung oder die mehreren Ausnehmungen können an einem Rand des Basisteils angeordnet sein. Besonders bevorzugt kann der Basisteil ein Vorsprung und eine Ausnehmung, insbesondere mehrere Vorsprünge und mehrere Ausnehmungen umfassen. Der Vorsprung und die Ausnehmung, insbesondere die mehreren Vorsprünge und die mehreren Ausnehmungen können an der proximalen Seite des Basisteils vorgesehen sein. Ferner können der Vorsprung und die Ausnehmung, insbesondere die mehreren Vorsprünge und die mehreren Ausnehmungen an dem Rand des Basisteils angeordnet sein. Besonderes bevorzugt kann der Basisteil eine Vorsprungs- und Ausnehmungsanordnung aufweisen. Die Ausnehmung, insbesondere die mehreren Ausnehmungen können Teil der Vorsprungs- und Ausnehmungsanordnung sein. Die Vorsprungs- und Ausnehmungsanordnung kann an der proximalen Seite des Basisteils vorgesehen sein. Die Vorsprungs- und Ausnehmungsanordnung kann an dem Rand des Basisteils vorgesehen sein. Vorzugsweise kann die Vorsprungs- und Ausnehmungsanordnung durchgehend ausgebildet sein.

Ferner kann der Basisteil eine Ringnut aufweisen. Die Ringnut kann an der distalen Seite des Basisteils angeordnet sein. Die Ringnut des Basisteils kann vorzugsweise durchgehend ausgebildet sein.

Der Basisteil der ersten und der zweiten Ausführungsform kann vorzugweise kreisförmig ausgebildet sein. Andere Ausgestaltungen können vorgesehen sein.

Der Basisteil der ersten und der zweiten Ausführungsform ist mit dem Kappenteil zum Halten des Infusionssets durch einen Patienten oder durch eine Insertionsvorrichtung oder Einstechhilfe verbindbar oder ist mit dem Konnektorteil mit einer Zuführleitung zur Verbindung mit einer Infusionsvorrichtung verbindbar, insbesondere drehbar verbindbar.

Der mit dem Kappenteil verbundene Basisteil der ersten und der zweiten Ausführungsform kann auf der Haut des Patienten angebracht oder entfernt werden.

Der Konnektorteil der ersten und der zweiten Ausführungsform kann vorzugsweise kreisförmig ausgebildet sein. Vorzugsweise kann der Konnektorteil kappenförmig ausgebildet sein. Ferner kann der Konnektorteil vorzugsweise elastisch ausgebildet sein. Andere Ausgestaltungen können vorgesehen sein.

Der Konnektorteil kann eine Abragung an der distalen Seite umfassen. Die Abragung kann an einem Rand des Konnektorteils vorgesehen sein. Die Abragung kann an einer Mantelinnenfläche des kappenförmigen Konnektorteils angeordnet sein. Die Abragung des Konnektorteils kann sich von der Mantelinnenfläche in einem beliebigen Winkel zur Drehachse, insbesondere radial nach innen erstrecken. Vorzugsweise können an dem Konnektorteil zwei Abragungen vorgesehen sein. Die zwei Abragungen können vorzugsweise um 180° zueinander versetzt an dem Konnektorteil angeordnet sein. Ferner kann an dem Konnektorteil ein Führungsschlitz zum Führen einer Kante des Basisteils vorgesehen sein. Der Führungsschlitz ist proximal von der Abragung an dem Konnektorteil angeordnet. An dem Konnektorteil können vorzugweise zwei Führungsschlitze vorgesehen sein. Besonders bevorzugt kann jeder Führungsschlitz eine Abragung aufweisen. Ein Greifelement kann an einer Mantelaussenfläche des Konnektorteils vorgesehen sein. Ferner kann vorzugsweise das Greifelement um 90° versetzt zu dem Führungsschlitz und/oder der Abragung oder zu den zwei Führungsschlitzen und/oder den zwei Abragungen an der Mantelaussenfläche des Konnektorteils vorgesehen sein. Das Greifelement kann vorzugsweise an dem Rand und an der Mantelaussenfläche des Konnektorteils angeordnet sein. Besonders bevorzugt kann der Konnektorteil zwei Greifelemente aufweisen. Das Greifelement kann dem besseren Greifen des Konnektorteils dienen. Das Greifelement kann vorzugsweise rippenförmig ausgebildet sein. Das Greifelement kann derart ausgebildet und/oder an dem Konnektorteil angeordnet sein, dass der Patient den Konnektorteil mit dem Basisteil verbinden, insbesondere verschnappen und der Konnektorteil von dem Basisteil lösen kann.

Um den Konnektorteil mit der Zuführleitung mit dem Basisteil lösbar zu verbinden, insbesondere zu verschnappen, kann der Konnektorteil auf den Basisteil gesetzt werden. Die an der Mantelinnenseite des Konnektorteils angeordnete Durchstechkanüle kann durch das Septum des Basisteils dringen, um eine fluide Verbindung zwischen dem Konnektorteil und dem Basisteil zu bilden. Der Basisteil und der Konnektorteil können miteinander drehbar verbunden sein, um das flüssige Medikament von der Infusionsvorrichtung an den Patienten zu verabreichen.

Der Konnektorteil kann mit dem Basisteil unkorrekt oder unvollständig verbunden, insbesondere verschnappt werden.

Die Abragung des Konnektorteils ist derart in einen Eingriff mit der Vorsprungs- und Ausnehmunganordnung bringbar, dass bei relativer Drehung zwischen dem Konnektorteil und dem Basisteil ein akustische und/oder ein taktiles Signal erzeugt wird, um anzuzeigen, dass der Konnektorteil unkorrekt oder unvollständig mit dem Basisteil verbunden ist. Bei relativer Drehung zwischen dem Konnektorteil und dem Basisteil kann die Abragung des Konnektorteils über die Vorsprungs- und Ausnehmungsanordnung des Basisteils gleiten. Eine unkorrekte oder eine unvollständige Verbindung, insbesondere Verschnappung kann das Risiko einer unzureichenden Medikamentenzufuhr bergen, welche von dem Konnektorteil über den Basisteil an den Patienten abgeben wird.

Der Konnektorteil kann mit dem Basisteil korrekt oder vollständig verbunden, insbesondere verschnappt werden.

Die Abragung des Konnektorteils ist derart in einen Eingriff mit der Ringnut des Basisteils bringbar, dass das Konnektorteil relativ zu dem Basisteil geräuschlos und/oder ohne taktiles Signal erzeugend drehbar ist, um anzuzeigen, dass der Konnektorteil korrekt oder vollständig mit dem Basisteil verbunden ist.

Ferner kann die Kante des Basisteils mit dem an dem Konnektorteil vorgesehenen Führungsschlitz, insbesondere mit den zwei an dem Konnektorteil vorgesehene Führungsschlitze in einen Eingriff gelangen. Der Kappenteil der ersten und der zweiten Ausführungsform kann vorzugsweise kreisförmig ausgebildet sein. Vorzugsweise kann der Kappenteil kappenförmig ausgebildet sein. Ferner kann der Kappenteil vorzugsweise elastisch ausgebildet sein. Andere Ausgestaltungen können vorgesehen sein.

Der Kappenteil kann einen Sperrzahn aufweisen. Der Sperrzahn kann an einem Rand des Kappenteils angeordnet sein. Der Sperrzahn des Kappenteils kann an einer Mantelinnenseite des kappenförmigen Kappenteils angeordnet sein. Der Sperrzahn des Kappenteils kann sich von der Mantelinnenfläche in einem beliebigen Winkel zur Drehachse, insbesondere radial nach innen erstrecken.

Der Sperrzahn kann derart geformt sein, dass er in die Ausnehmung, insbesondere in eine Ausnehmung der mehreren Ausnehmungen des Basisteils passt. Der Sperrzahn des Kappenteils ist derart mit der Ausnehmung, insbesondere mit einer Ausnehmung der mehreren Ausnehmungen des Basisteils in einen Eingriff, insbesondere in einen Formeingriff bringbar, um eine verdrehsichere Verbindung zwischen dem Basisteil und dem Kappenteil zu bilden.

Vorzugsweise können an dem Kappenteil zwei Sperrzähne vorgesehen sein. Die zwei Sperrzähne können vorzugsweise um 180° zueinander versetzt an dem Kappenteil angeordnet sein. Ferner kann an dem Kappenteil eine Führungsnut zum Befestigung der Kante des Basisteils vorgesehen sein. Die Führungsnut kann distal von dem Sperrzahn an dem Kappenteil angeordnet sein. An dem Kappenteil können vorzugweise zwei Führungsnuten vorgesehen sein. Besonders bevorzugt kann an jeder Führungsnut ein Sperrzahn angeordnet sein. Besonders bevorzugt kann der Kappenteil ein Halteelement aufweisen. Das Halteelement kann vorzugsweise an einer Mantelaussenfläche des Kappenteils angeordnet sein. Das Halteelement kann sich von der Mantelaussenfläche des Kappenteils vorzugsweise in die proximale Richtung erstrecken. Besonders bevorzugt können an der Mantelaussenfläche des Kappenteils zwei parallel angeordnete Halteelemente vorgesehen sein. Das Halteelement kann vorzugsweise ungefähr in der axialen Flucht zu der Führungsnut und/oder dem Sperrzahn angeordnet sein. Das Halteelement kann derart ausgebildet und/oder an dem Kappenteil angeordnet sein, dass der Patient den Kappenteil mit dem Basisteil verbinden, insbesondere verschnappen und den Kappenteil von dem Basisteil lösen kann. Besonders bevorzugt kann der Kappenteil einen Griffteil umfassen. Das Griffteil kann vorzugsweise an der Mantelaussenfläche des Kappenteils und in der Mitte des Kappenteils angeordnet sein. Der Griffteil kann sich von der Mantelaussenfläche des Kappenteils vorzugsweise in die proximale Richtung erstrecken. In der ersten Ausführungsform des Infusionssets kann der Griffteil die steife Nadel halten, wobei sich die steife Nadel von der Mantelinnenfläche des Kappenteils in die distale Richtung erstrecken kann. Der Griffteil kann ferner dazu dienen den mit dem Basisteil verbundenen, insbesondere verschnappten Kappenteil in eine Insertionsvorrichtung oder in eine Einstechhilfe zu setzen. Die weiche oder die steife Kanüle des Infusionssets kann mittels der Insertionsvorrichtung oder Einstechhilfe direkt in die Haut platziert werden und mit dem Heftpflaster an Ort und Stelle gehalten werden. Wenn der Kappenteil mit dem Basisteil verbunden, insbesondere verschnappt ist, kann der Sperrzahn des Kappenteils in die Ausnehmung der Vorsprungs- und Ausnehmungsanordnung des Basisteils eingreifen, um eine Verdrehsicherung zu bilden. Durch den Eingriff zwischen dem Sperrzahn des Kappenteils und der Ausnehmung des Basisteils kann verhindert werden, dass der Kappenteil relativ zu dem Basisteil drehen kann. Durch die Verdrehsicherung zwischen dem Kappenteil und dem Basisteil kann der Patient die Schutzfolie des Heftpflasters besser von dem Basisteil entfernen. Besonders bevorzugt kann eine erste Verdrehsicherung zwischen dem Griffteil des Kappenteils und einer Aufnahme der Insertionsvorrichtung oder der Einstechhilfe ausgebildet sein und eine zweite Verdrehsicherung kann zwischen dem Sperrzahn des Kappenteils und der Ausnehmung des Basisteils ausgebildet sein, wobei die beiden Verdrehsicherungen dazu dienen können, dass der Patient die Schutzfolie des Heftpflasters besser von dem Basisteil entfernen kann, wenn das Infusionsset in der Insertionsvorrichtung oder in der Einstechhilfe eingesetzt ist.

Der Kappenteil kann ferner zwischen dem Griffteil und dem Halteelement einen Schlitz in dem Kappenteil aufweisen. Der Schlitz kann vorzugsweise parallel zu dem Halteelement und/oder zu der Führungsnut des Kappenteils angeordnet sein. Der Schlitz kann durchgängig oder undurchgängig ausgebildet sein. Der Schlitz kann der besseren Befestigung des Kappenteils an dem Basisteil und zum besseren Lösen des Kappenteils von dem Basisteil dienen.

Die Erfindung wird im Folgenden anhand mehreren Figuren beschrieben. Die hierbei offenbarten Merkmale bilden je einzeln und in jeglicher Merkmalskombination die Erfindung vorteilhaft weiter.
Es zeigen:
Figur 1 eine Explosionsansicht der ersten Ausführungsform des erfindungsgemässen Infusionssets, wobei ein Kappenteil und ein Basisteil des Infusionssets ersichtlich sind.
Figur 2 eine Längsschnittansicht des Infusionssets gemäss Figur 1, wobei der Kappenteil mit dem Basisteil verbunden, insbesondere verschnappt ist.
Figur 3 eine Längsschnittansicht des Infusionssets gemäss Figur 1, wobei ein Konnektorteil mit dem Basisteil verbunden, insbesondere verschnappt ist.
Figur 4 eine Explosionsansicht der zweiten Ausführungsform des erfindungsgemässen Infusionssets wobei ein Kappenteil und ein Basisteil des Infusionssets ersichtlich sind.
Figur 5 eine Längsschnittansicht des Infusionssets gemäss Figur 4, wobei ein Kappenteil mit dem Basisteil verbunden, insbesondere verschnappt ist.
Figur 6 eine Längsschnittansicht des Infusionssets gemäss Figur 4 wobei ein Konnektorteil mit dem Basisteil verbunden, insbesondere verschnappt ist.
Figur 7 eine Draufsicht auf den Basisteil der ersten und der zweiten Ausführungsform des Infusionssets.
Figur 8 eine Längsschnittansicht eines Rands des Basisteils des Infusionssets entlang der Schnittlinie A-A gemäss Figur 1.
Figur 9 eine Längsschnittansicht des Kappenteils des Infusionssets gemäss Figur 1.
Figur 10 eine Perspektivenansicht des in der Figur 9 gekennzeichneten Ausschnitts des Kappenteils.
Figur 11 ist eine Seitenansicht des Konnektors der ersten und der zweiten Ausführungsform des Infusionssets.
Figur 12 ist eine Längsschnittansicht des Konnektors entlang der Schnittlinie B-B gemäss Figur 11.
Figur 13 eine Perspektivenansicht des Konnektors gemäss Figur 12.

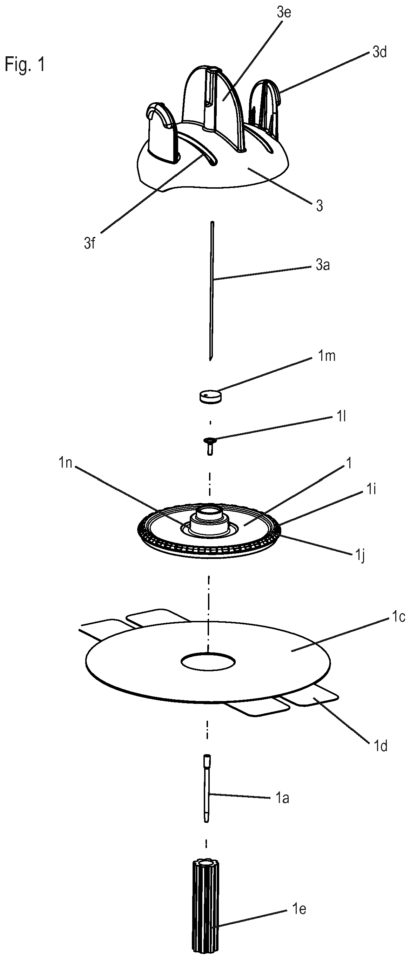

In der Figur 1 ist eine Explosionsansicht eines Infusionssets gemäss einer ersten Ausführungsform der vorliegenden Erfindung dargestellt, wobei ein Kappenteil und ein Basisteil der ersten Ausführungsform des Infusionssets ersichtlich sind.

Das Infusionsset umfasst einen kreisförmigen und kappenförmigen Kappenteil (3) mit einer steifen Nadel (3a). Der Kappenteil (3) ist elastisch ausgebildet. Die steife Nadel (3a) kann vorzugsweise aus Stahl oder aus einem alternativen Material gebildet sein. Die Nadel (3a) ist mit dem Kappenteil (3) fest verbunden. An einer Mantelaussenfläche des Kappenteils (3) ist ein, insbesondere zwei Halteelemente (3d) und ein Griffteil (3e) vorgesehen. Das Halteelement (3d) dient dem Halten des Infusionssets und der Griffteil (3e) dient dem Einsetzen des Infusionssets in eine Insertionsvorrichtung oder in eine Einstechhilfe. Ferner ist in dem Kappenteil (3) ein, insbesondere zwei durchgängige Schlitze (3f) vorgesehen. Ein proximales Ende der steifen Nadel (3) ist mit dem Griffteil (3e) des Kappenteils (3) verbunden.

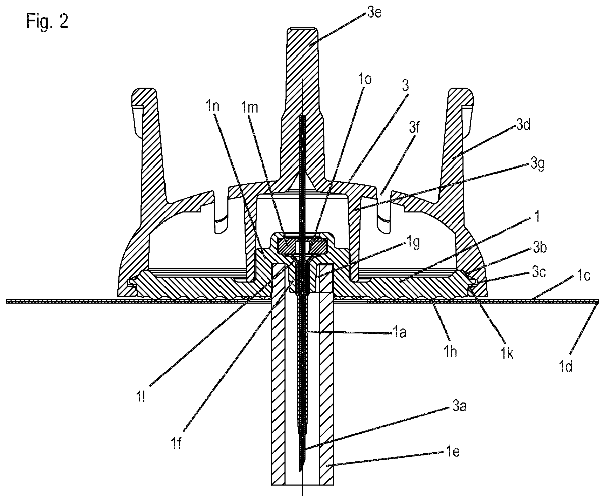

Ferner umfasst das Infusionsset einen kreisförmigen, insbesondere flachen Basisteil (1). Zum Schutz vor Stichverletzungen durch die Nadel (3a) kann an dem Basisteil (1) ein Schutzelement (1e) vorgesehen sein. An einer proximalen Seite und am Rand des Basisteils (1) sind mehrere Vorsprünge (1i) und mehrere Ausnehmungen (1j) angeordnet. Der Basisteil umfasst ferner einen Basiszylinder (1n), welcher zylinderförmig ausgebildet ist. Der Basiszylinder (1n) ist auf der proximalen Seite des Basisteils angeordnet und ragt in die proximale Richtung ab. In dem Basiszylinder ist ein Durchgang (Figur 2; 1o) vorgesehen. Ein "eyelet" (1l) ist in dem Durchgang (Figur 2; 1o) des Basiszylinder (1n) eingesetzt, um eine weiche Kanüle (1a) mit dem Basisteil (1) zu verbinden. Die weiche Kanüle (1a) ist vorzugsweise aus einem weichen Material gebildet. Der Durchgang (Figur 2; 1o) wird mit einem Septum (1m) verschlossen, wobei das Septum (1m) an dem proximalen Ende des "eyelet" (1l) angeordnet ist. An einer distalen Seite des Basisteils sind Nocken (Figur 2; 1h) vorgesehen. Die Nocken (Figur 2; 1h) sind mit einem Heftpflaster (1c) des Basisteils (1) verschmolzen, sodass das Heftpflaster an dem Basisteil (1) verbunden ist. Das Heftpflaster (1c) dient dem Befestigen des Infusionssets auf der Haut eines Patienten. Eine distale Seite des Heftpflasters (1c) kann mit einer Schutzfolie (1d) bedeckt sein. Vor dem Anbringen des Infusionssets auf der Haut des Patienten, nimmt der Patient die Schutzfolie (1d) von dem Haftpflaster (1c) ab.

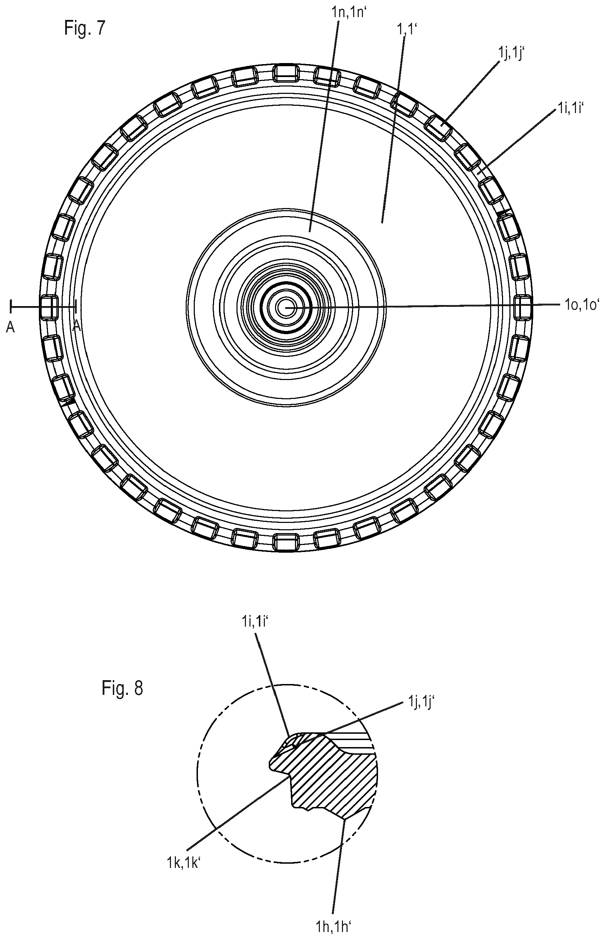

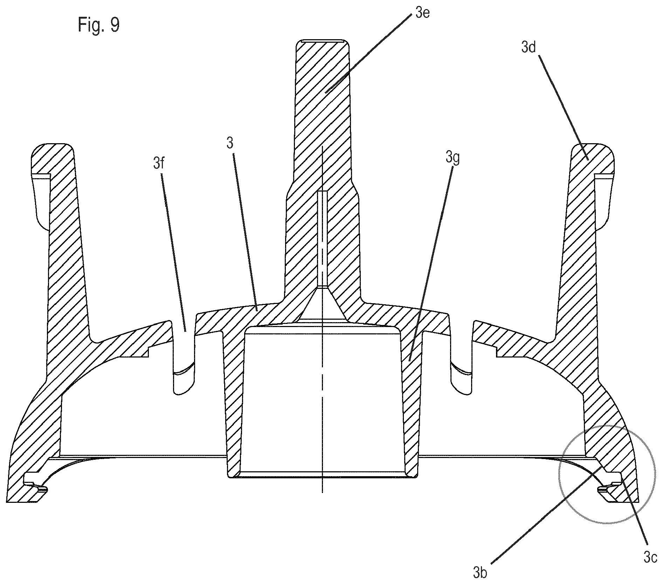

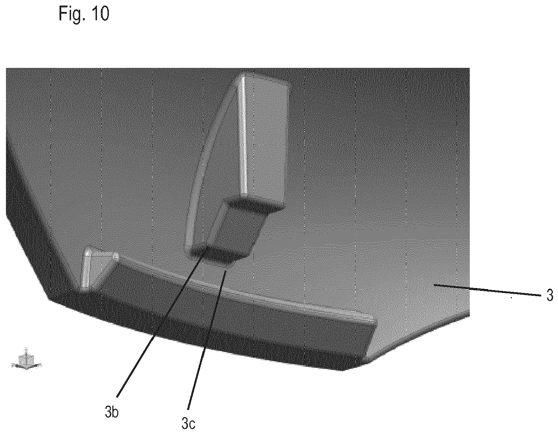

In der Figur 2 ist eine Längsschnittansicht des Infusionssets gemäss Figur 1 dargestellt, wobei der Kappenteil mit dem Basisteil verbunden, insbesondere verschnappt ist. Die steife Nadel (3a) des Kappenteils ist in der weichen Kanüle (1a) des Basisteils derart aufgenommen, dass ein distales Ende der Nadel (3a) des Kappenteils über ein distales Ende der weichen Kanüle (1a) des Basisteils ragt. Die konzentrische Anordnung der weichen Kanüle (1a) und der steifen Nadel (3a) dient der Führung der weichen Kanüle (1a), wenn die weiche Kanüle (1a) des Basisteils (1) in die Haut des Patienten eingestochen wird. Die weiche Kanüle (1a) ist mit dem "eyelet" (1l) kraftschlüssig verbunden, insbesondere verpresst und in dem Durchgang (1o) des Basiszylinders (1n) eingepresst. Das Schutzelement (1e) ist in einer an einer distalen Seite des Basiszylinders (1n) des Basisteils (1) vorgesehenen Öffnung (1g) lösbar eingepresst. Der Kappenteil (3) ist mit dem Basisteil (1) verschnappt. Der Rand des Kappenteils (3) weist eine Führungsnut (3c) auf, welcher eine Kante des Basisteils (1) aufnimmt. Ferner ragt ein Sperrzahn (3b), welcher an einer Mantelinnenfläche des Kappenteils (3) vorgesehen ist, in eine Ausnehmung (1j), welche an der distalen Seite des Basisteils (1) angeordnet ist. Wie in der Figur 7 und in der Figur 8 ersichtlich ist, ist die Ausnehmung (1j) an dem Rand des Basisteils (1) angeordnet. Die Ausnehmung (1j) ist ferner ein Teil einer Vorsprungs- (1i) und Ausnehmungsanordnung (1j) des Basisteils (1). Wie in der Figur 9 und in der Figur 10 ersichtlich ist, ragt der Sperrzahn (3b) des Kappenteils (3) von der Mantelinnenseite des Kappenteils (3) radial nach innen. Der Sperrzahn (3b) des Kappenteils (3) ist derart in einen Eingriff, insbesondere in einem Formeingriff mit der Ausnehmung (1j) des Basisteils (1) bringbar, um eine verdrehsichere Verbindung zwischen dem Basisteil (1) und dem Kappenteil (3) zu bilden. Durch die Verdrehsicherung zwischen dem Kappenteil (3) und dem Basisteil (1) kann der Patient die Schutzfolie (1d) des Heftpflasters (1c) besser von dem Basisteil (1) entfernen.

Um das Infusionsset auf der Haut des Patienten anzubringen, nimmt der Patient das Schutzelement (1e) von dem Basisteil (1) ab. Danach zieht der Patient die Schutzfolie (1d) von dem Heftpflaster (1c) ab. Der mit dem Basisteil (1) verschnappte Kappenteil (3) wird auf die Haut des Patienten gesetzt. Dabei dringt die weiche Kanüle (1a) des Basisteils (1) mit der darin aufgenommenen steifen Nadel (3a) des Kappenteils durch die Haut bis in das subkutane Gewebe ein. Die Nocken (1h) des Basisteils (1) beabstanden den Basisteil (1) von der Haut des Patienten und dienen der Luftzirkulation zwischen dem Basisteil und der Haut des Patienten. Danach wird der Kappenteil (3) von dem Basisteil (1) abgenommen, indem der Patient die beiden Halteelemente (3d) nach innen vorspannt, wobei die Schnappverbindung zwischen dem Kappenteil (3) und dem Basisteil (1) gelöst wird. Das Schutzelement (1e) kann vorzugsweise über die an dem Kappenteil (3) vorgesehenen steifen Nadel (3a) gesetzt werden, um den Patienten vor Stichverletzungen zu schützen. Besonders bevorzugt kann hierfür eine an der Mantelinnenfläche des Kappenteils (3) vorgesehene Halterung vorgesehen sein.

Danach wird ein Konnektorteil (2) mit einem Schlauch (2d) auf den Basisteil (1) angebracht werden. Der Schlauch (2d) umfasst einen Adapter zur lösbaren Befestigung an eine Infusionsvorrichtung, insbesondere an eine Infusionspumpe. Der Schlauch (2d) des Konnektorteils (2) ist mit dem Adapter mittels einem Klebstoff verbunden, welcher durch Bestrahlung mit UV-Licht aushärtet.

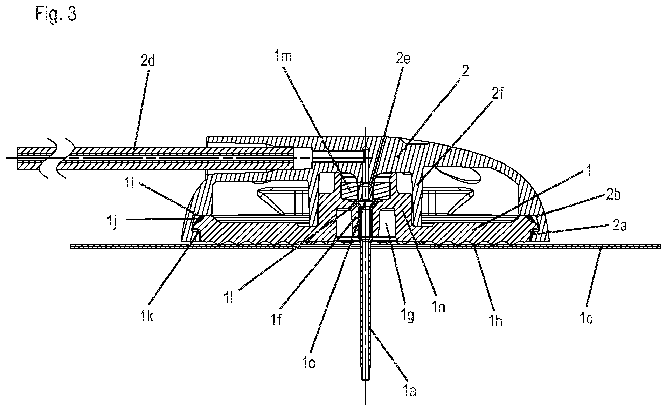

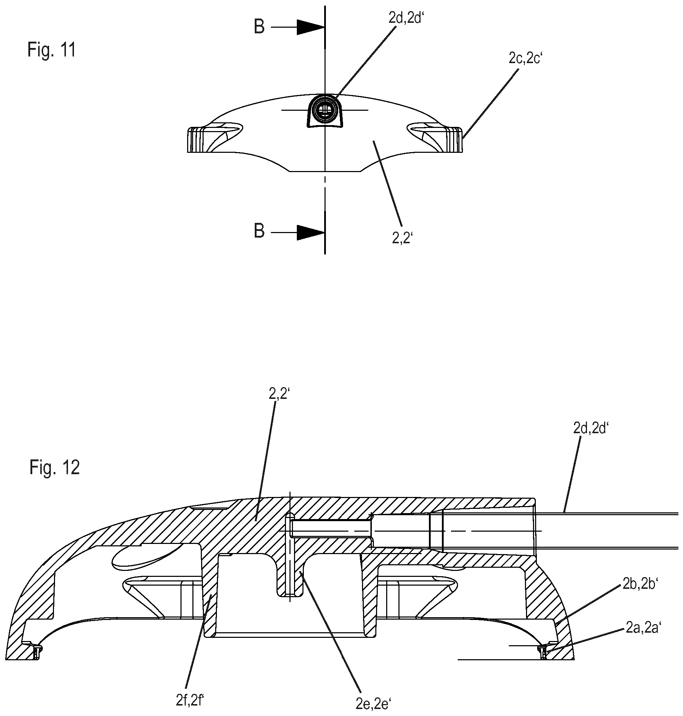

In der Figur 3 ist eine Längsschnittansicht des Infusionssets gemäss Figur 1 dargestellt, wobei ein Konnektorteil mit dem Basisteil verbunden, insbesondere verschnappt ist. Der Schlauch (2d) des Konnektorteils (2) ist mittels einem Klebstoff, welcher durch Bestrahlung mit UV-Licht aushärtet, mit dem Konnektorteil (2) verbunden. Der Schlauch (2d) ist in fluider Verbindung mit einer an einer Mantelinnenfläche angeordneten Durchstechkanüle (2e). Vorzugsweise ist der Schlauch (2d) um 90° versetzt zu der Durchstechkanüle (3e) in dem Konnektorteil angeordnet, wie in der Figur 12 gezeigt ist. Die Durchstechkanüle (2e) des Konnektorteils (2) dringt durch das an dem Basisteil (1) vorgesehene Septum (1m) und ragt vorzugsweise in das "eyelet" (1l), insbesondere in den proximalen Teil des "eyelet" (1l) des Basisteils (1). Ein an der Mantelinnenfläche des Konnektorteils (2) vorgesehener Konnektorzylinder (2f) nimmt den in die proximale Richtung ragenden Basiszylinder (1n) des Basisteils (1) auf. Der Konnektorzylinder (2f) ist mit dem Basiszylinder (1n) in einem Führungseingriff, insbesondere in einem Gleiteingriff, wobei der Konnektorteil (2) relativ zu dem Basisteil (1) drehbar, insbesondere um einen beliebigen Winkel zueinander drehbar ist. Das abzugebende Medikament wird von der Infusionsvorrichtung über den Konnektorteil (2) und den Basisteil (1) in den Körper des Patienten verabreicht.

Bei korrekter oder vollständiger Verschnappung zwischen dem Konnektorteil (2) und dem Basisteil (1), wie in der Figur 3 gezeigt ist, gelangt die Kante des Basisteils (1) in einen an dem Konnektorteil (2) vorgesehenen Führungsschlitz (2b). Ferner ragt eine an der Mantelinnenseite des Konnektorteils (2) angeordnete Abragung (2a) in eine an dem Basisteil (1) vorgesehene Ringnut (1k). Die Abragung (2a) des Konnektorteils (2) ist in Eingriff mit der Ringnut (1k) des Basisteils (1). Bei relativer Drehung zwischen dem Konnektorteil (2) und dem Basisteil (1) gleitet die Abragung (2a) des Konnektorteils (2) geräuschlos und/oder ohne taktiles Signal erzeugend in der Ringnut (1k) des Basisteils (1).

Bei unkorrekter oder unvollständiger Verbindung, insbesondere Verschnappung zwischen dem Konnektorteil (2) gelangt die Abragung (2a) des Konnektorteils (2) in die Vorsprungs- (1i) und Ausnehmungsanordnung (1j) des Basisteils (1). Die Abragung (2a) des Konnektorteils (2) ist in Eingriff mit der Vorsprungs-(li) und Ausnehmungsanordnung (1j) des Basisteils (1). Bei relativer Drehung zwischen dem Konnektorteil (2) und dem Basisteil (1) gleitet die Abragung (2a) des Konnektorteils (2) über die Vorsprungs- (1i) und Ausnehmungsanordnung (1j) des Basisteils (1). Bei relativer Drehung zwischen dem Konnektorteil (2) und dem Basisteil (1) wird vorzugsweise ein akustisches und/oder ein taktiles Signal erzeugt, um anzuzeigen, dass der Konnektorteil (2) nicht korrekt oder nicht vollständig mit dem Basisteil (1) verbunden, insbesondere verschnappt ist. Eine unkorrekte oder eine unvollständige Verbindung, insbesondere Verschnappung birgt das Risiko einer unzureichenden Medikamentenzufuhr, welche von dem Konnektorteil über den Basisteil an den Patienten abgeben wird.

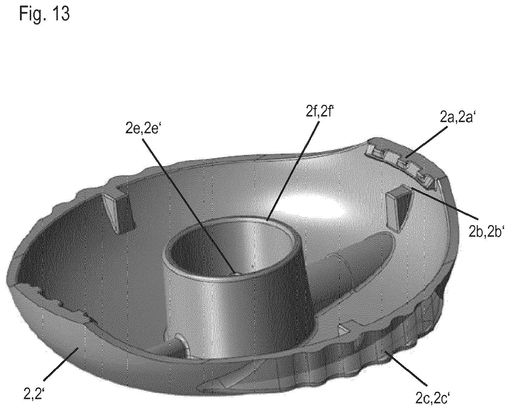

Um den Konnektorteil (2) von dem Basisteil (1) zu lösen, spannt der Patient zwei an dem Konnektorteil (2) vorgesehene Greifelemente (2c), wie in der Figur 11 und in der Figur 13 dargestellt ist, nach innen. Die Schnappverbindung zwischen dem Konnektorteil (2) und dem Basisteil (1) wird dabei gelöst.

Um den Basisteil (1) von der Haut des Patienten abzunehmen, löst der Patient das Heftpflaster (1c) von der Haut des Patienten und nimmt das Basisteil (1) von der Haut weg. Alternativ kann der Patient den Basisteil (1) mit einem Kappenteil, welcher keine steife Nadel (3a) aufweist verbinden, wobei der Patient danach den mit dem Basisteil (1) verbundenen Kappenteil (3) aus der Haut des Patienten ziehen kann und das Infusionsset entsorgen kann.

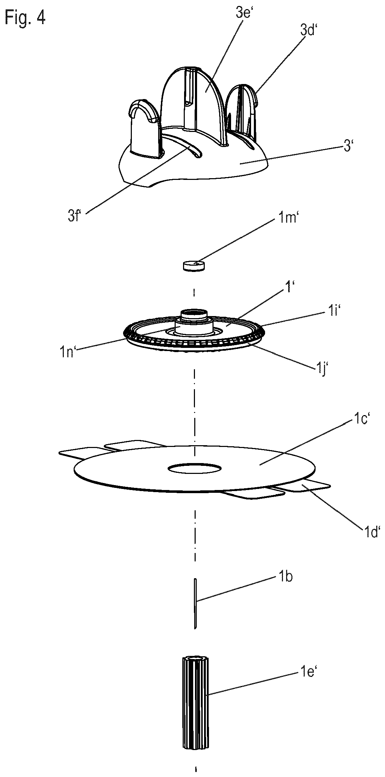

In der Figur 4 ist eine Explosionsansicht einer zweiten Ausführungsform des erfindungsgemässen Infusionssets gezeigt, wobei ein Kappenteil (3') und ein Basisteil (1') des Infusionssets ersichtlich ist.

Das Infusionsset weist einen kreisförmigen und kappenförmigen Kappenteil (3') auf. Der Kappenteil (3') umfasst ein, insbesondere zwei Halteelemente (3d') und ein Griffteil (3e'), welche an einer Mantelaussenfläche des Kappenteils (3') angebracht sind. Ferner kann in dem Kappenteil (3') ein, insbesondere zwei durchgängige Schlitze (3f') vorgesehen sein.

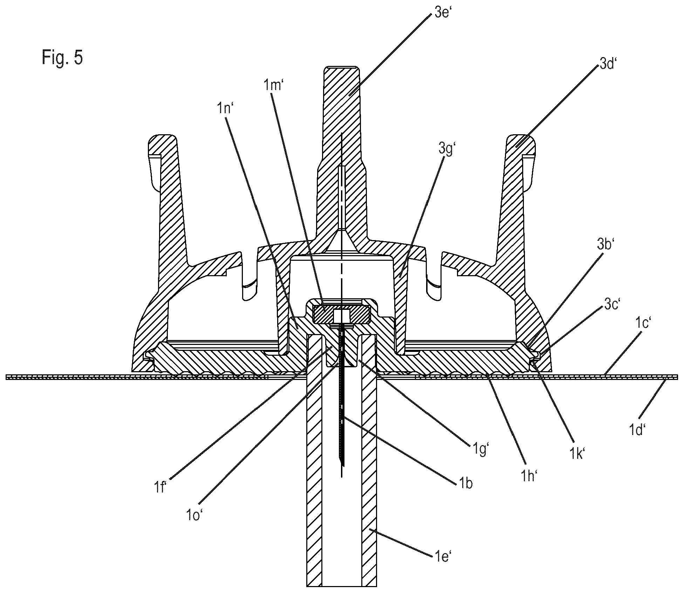

Das Infusionsset weist ferner einen kreisförmigen, insbesondere flachen Basisteil (1') auf. In einem Durchgang (Figur 5; 1o') eines Basiszylinders (1n') des Basisteils (1') ist eine steife Kanüle (1b') befestigt. Zur Befestigung der steifen Kanüle (1b') in dem Basisteil (1') wird zwischen der steifen Kanüle (1b') und dem Kanülenzylinder (1f') ein Klebstoff angebracht, insbesondere ein Klebstoff, welcher durch Bestrahlung mit UV-Licht aushärtet. Zum Schutz vor Stichverletzungen durch die steife Kanüle (1b') kann an dem Basisteil (1') ein Schutzelement (1e') vorgesehen sein. An einer proximalen Seite und am Rand des Basisteils (1) sind mehrere Vorsprünge (1i) und mehrere Ausnehmungen (1j) angeordnet. Der Durchgang (Figur 5; 1o') wird mit einem Septum (1m') verschlossen, wobei das Septum (1m) an einem proximalen Ende der steifen Kanüle (1b') vorgesehen ist. An einer distalen Seite des Basisteils sind Nocken (Figur 5; 1h') angeordnet. Die Nocken (Figur 5; 1h) sind mit einem Heftpflaster (1c') des Basisteils (1') verschmolzen, sodass das Heftpflaster an dem Basisteil (1') befestigt ist. Das Heftpflaster (1c) dient dem Befestigen des Infusionssets auf der Haut eines Patienten. Eine distale Seite des Heftpflasters (1c) kann mit einer Schutzfolie (1d') bedeckt sein. Vor dem Anbringen des Infusionssets auf der Haut des Patienten, nimmt der Patient die Schutzfolie (1d') von dem Haftpflaster (1c') ab.

In der Figur 5 ist eine Längsschnittansicht des Infusionssets gemäss Figur 4 dargestellt, wobei der Kappenteil (3') mit dem Basisteil (1') verbunden, insbesondere verschnappt ist. Die zweite Ausführungsform des Infusionssets unterscheidet sich von der ersten Ausführungsform dadurch, dass der Kappenteil (3') keine steife Nadel aufweist. Ferner ist anstatt einer weichen Kanüle die steife Kanüle (1b') in dem Basisteil (1') befestigt. Das Septum (1m') verschliesst das proximale Ende der steifen Kanüle (1b'), welche mit dem Basiszylinder (1n') verklebt ist. Aufgrund der Klebeverbindung zwischen steifer Kanüle (1b') und dem Basiszylinder (1n') kann auf ein Verbindungselement, insbesondere auf ein "eyelet" verzichtet werden.

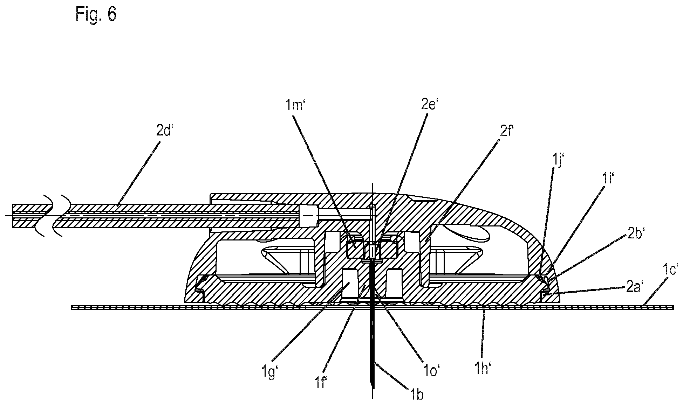

In der Figur 6 ist eine Längsschnittansicht des Infusionssets gemäss Figur 4 dargestellt, wobei ein Konnektorteil (2') mit dem Basisteil (1') verbunden, insbesondere verschnappt ist. Die zweite Ausführungsform des Infusionssets unterscheidet sich von der ersten Ausführungsform dadurch, dass eine an dem Konnektorteil (3') vorgesehene Durchstechkanüle (2e') durch das Septum (1m') des Basisteils (1') dringt und eine Fluidverbindung zum Basisteil (1') bildet.

### Bezugszeichen:

- 1; 1': Basisteil
- 1a: weiche Kanüle
- 1b: steife Kanüle
- 1c; 1c': Heftpflaster
- 1d; 1d': Schutzfolie
- 1e; 1e': Schutzelement
- 1f; 1f': Kanülenzylinder
- 1g; 1g': Öffnung
- 1h; 1h': Nocke
- 1i; 1i': Vorsprung
- 1j; 1j': Ausnehmung
- 1k; 1k': Ringnut
- 1l: eyelet
- 1m; 1m': Septum
- 1n; 1n': Basiszylinder
- 1o; 1o': Durchgang
- 2, 2': Konnektorteil
- 2a; 2a': Abragung
- 2b; 2b': Führungsschlitz
- 2c; 2c': Greifelement
- 2d; 2d': Schlauch
- 2e; 2e': Durchstechkanüle
- 2f; 2f': Konnektorzylinder
- 3; 3': Kappenteil
- 3a: steife Nadel
- 3b; 3b': Sperrzahn
- 3c; 3c': Führungsnut
- 3d; 3d': Halteelemente
- 3e; 3e': Griffteil
- 3f; 3f': Schlitz
- 3g; 3g': Kappenzylinder

## Patentansprüche

1. Infusionsset mit
- einem Basisteil (1; 1'), wobei der Basisteil mit einem Kappenteil (3; 3') zum Halten des Infusionssets durch einen Patienten oder durch eine Insertionsvorrichtung oder Einstechhilfe verbindbar ist oder mit einem Konnektorteil (2, 2') mit einer Zuführleitung zur Verbindung mit einer Infusionsvorrichtung verbindbar ist, wobei der Basisteil (1, 1') eine proximale Seite und eine distale Seite aufweist und wobei an der distalen Seite des Basisteils (1, 1') eine Kanüle (1a; 1b) zur Zuführung eines flüssigen Medikaments in den Körper des Patienten vorgesehen ist und wobei ferner an der distalen Seite eine Nocke (1h, 1h'), insbesondere mehrere Nocken (1h, 1h') vorgesehen sind,
- einem Heftpflaster (1c, 1c') zur Befestigung des Infusionssets auf der Haut des Patienten, **dadurch gekennzeichnet, dass** die Nocke (1h, 1h'), insbesondere die mehreren Nocken (1h, 1h') mit dem Heftpflaster verbunden, insbesondere verschmolzen sind.

2. Infusionsset nach einem der vorhergehenden Ansprüche, **dadurch gekennzeichnet, dass** die Nocke (1h, 1h'), insbesondere die Nocken (1h, 1h') durch ein Ultraschallschweissen mittels einer Sonotrode mit dem Heftpflaster (1c, 1c') verschmolzen sind.

3. Infusionsset nach Anspruch 1 oder 2, **dadurch gekennzeichnet, dass** die Nocke (1h, 1h'), insbesondere die mehreren Nocken (1h, 1h') etwa pyramidenförmig oder kegelförmig ausgebildet sind.

4. Infusionsset nach einem der vorhergehenden Ansprüchen, **dadurch gekennzeichnet, dass** die Nocken (1h, 1h') auf der distalen Seite des Basisteils (1, 1') gleichmässig oder ungleichmässig verteilt angeordnet sind.

5. Verfahren zur Herstellung eines Infusionssets mit den folgenden Schritten:
- Bereitstellen eines Basisteils (1; 1') wobei der Basisteil mit einem Kappenteil (3; 3') zum Halten des Infusionssets durch einen Patienten oder durch eine Insertionsvorrichtung oder Einstechhilfe verbindbar ist oder mit einem Konnektorteil (2, 2') mit einer Zuführleitung zur Verbindung mit einer Infusionsvorrichtung verbindbar ist, wobei der Basisteil (1,1') eine proximale Seite und eine distale Seite aufweist und wobei an der distalen Seite des Basisteils (1, 1') eine Kanüle (1a; 1b) zur Zuführung eines flüssigen Medikaments in den Körper des Patienten vorgesehen ist und wobei ferner an der distalen Seite eine Nocke (1h, 1h'), insbesondere mehrere Nocken (1h, 1h') vorgesehen sind,
- Bereitstellen eines Heftpflaster (1c, 1c') zur Befestigung des Infusionssets auf der Haut des Patienten, wobei
- der Basisteil (1, 1') mit der Nocke (1, 1h'), insbesondere mit den mehreren Nocken (1h, 1h') mit dem Heftpflaster verbunden, insbesondere verschmolzen wird.

6. Verfahren nach einem der Ansprüche 5, **dadurch gekennzeichnet, dass** der Nocke (1h, 1h'), insbesondere die Nocken (1h, 1h') durch ein Ultraschallschweissen mittels einer Sonotrode mit dem Heftpflaster (1c, 1c') verschmolzen werden.

7. Verfahren nach Anspruch 5 oder 6, **dadurch gekennzeichnet, dass** die Nocke (1h, 1h'), insbesondere die mehreren Nocken (1h, 1h') etwa pyramidenförmig oder kegelförmig ausgebildet sind.

8. Verfahren nach einem der Ansprüche 5 bis 7, **dadurch gekennzeichnet, dass** die Nocken (1h, 1h') auf der distalen Seite des Basisteils (1, 1') gleichmässig oder ungleichmässig verteilt angeordnet sind.
